# EUROPÄISCHE PATENTSCHRIFT

(11) **EP 4 119 926 B1**
(45) Veröffentlichungstag und Bekanntmachung des Hinweises auf die Patenterteilung: **14.05.2025**
(21) Anmeldenummer: 22181356.1
(22) Anmeldetag: 27.06.2022
(51) Int. Cl.: G01N 17/00, G01N 33/44

(54) **ULTRASCHNELLE ALTERUNGSLÖSUNG**
ULTRA-FAST AGING SOLUTION
SOLUTION DE VIEILLISSEMENT ULTRARAPIDE

(30) Priorität: 15.07.2021 DE 102021207535
(43) Veröffentlichungstag der Anmeldung: 18.01.2023
(73) Patentinhaber: ContiTech Techno-Chemie GmbH, 61184 Karben (DE)
(72) Erfinder: Richards, Justin, 30165 Hannover (DE); Stuhrmann, Dirk, 30165 Hannover (DE); Dahn, Ulrich, 80636 München (DE); Schauberger, Daniel, 80636 München (DE)
(74) Vertreter: Continental Corporation

(56) Entgegenhaltungen:
- CN-A- 107 044 953
- BREDÁCS M. ET AL: "Accelerated aging of polyethylene pipe grades in aqueous chlorine dioxide at constant concentration", POLYMER DEGRADATION AND STABILITY, vol. 157, November 2018 (2018-11-01), GB, pages 80 - 89, XP055978573, ISSN: 0141-3910, DOI: 10.1016/j.polymdegradstab.2018.09.019
- ROY P K ET AL: "Accelerated aging of LDPE films containing cobalt complexes as prooxidants", POLYMER DEGRADATION AND STABILITY, BARKING, GB, vol. 91, no. 8, August 2006 (2006-08-01), pages 1791 - 1799, XP027949789, ISSN: 0141-3910, [retrieved on 20060801]
- FRANCESCO RECUPERO ET AL: "Free Radical Functionalization of Organic Compounds Catalyzed by N- Hydroxyphthalimide †", CHEMICAL REVIEWS, vol. 107, no. 9, September 2007 (2007-09-01), pages 3800 - 3842, XP055203517, ISSN: 0009-2665, DOI: 10.1021/cr040170k
- W. YU ET AL: "Deterioration of polyethylene pipes exposed to water containing chlorine dioxide", POLYMER DEGRADATION AND STABILITY, vol. 96, no. 5, May 2011 (2011-05-01), pages 790 - 797, XP055050277, ISSN: 0141-3910, DOI: 10.1016/j.polymdegradstab.2011.02.009
- AMORATI RICCARDO ET AL: "Hydroxylamines as Oxidation Catalysts:? Thermochemical and Kinetic Studies", THE JOURNAL OF ORGANIC CHEMISTRY, vol. 68, no. 5, February 2003 (2003-02-01), pages 1747 - 1754, XP055979242, ISSN: 0022-3263, Retrieved from the Internet <URL:https://pubs.acs.org/doi/pdf/10.1021/jo026660z> DOI: 10.1021/jo026660z

## Beschreibung

Die Erfindung betrifft ein Verfahren zur beschleunigten Alterung von Kunststoffartikeln mit einer flüssigen Mischung, die Verwendung der flüssigen Mischung zur beschleunigten Alterung von Kunststoffartikeln sowie eine flüssige Mischung zur beschleunigten Alterung von Kunststoffartikeln.

Kunststoffartikel werden zunehmend als Ersatz für metallische Werkstoffe für Anwendungen mit hoher Temperaturbelastung, z.B. in der Automobilindustrie, ins Auge gefasst, um Gewicht einzusparen. So können beispielweise Polyamide für Getriebe als Ölwannen und Gehäuse für Getriebesteuerungen, Anlaufscheiben und Magnetspulen, Fahrzeugtanks und für diverse Schläuche und Hüllen im Tanksystem verwendet werden.

Bei der Entwicklung neuer Kunststoffprodukte werden Prüfungen hinsichtlich der Alterungseigenschaften basierend auf Kundenanforderungen durchgeführt. Diese umfassen Material-, Halbzeug- und Produktprüfungen. Die Alterungsanforderungen der Kunden reichen von dynamischen Pulsationsversuchen hin zu statischen oder auch dynamischen Langzeitalterungen. Diese Prüfungen sind ein Teil der Entwicklung der Produkte. Die Produkte im finalen Design müssen diese Prüfungen bestehen, um den Kundenforderungen gerecht zu werden.

Die Alterungseigenschaften der eingesetzten Materialien aber auch der Produkte werden in der Entwicklungsphase evaluiert, um notwendige Anpassungen durchzuführen, falls die geforderten Eigenschaften nicht erreicht werden.

Nach dem Stand der Technik beläuft sich Dauer der Alterungsversuche auf 500-5000h. Diese Versuche sind wie oben beschrieben an den fertigen Produkten durchzuführen. Bei solchen Langzeittests werden die Bauteile Bedingungen unterworfen, denen sie im Einsatz ausgesetzt sind, z.B. über einen Zeitraum von 3000 Stunden. Anschließend wird geprüft, ob die gealterten Proben die geforderten Eigenschaften erfüllen. Aktuell existiert keine Möglichkeit, im Vorfeld eine Abschätzung über das Bestehen solcher Prüfungen abzugeben. Hinzukommt, dass neue Materialien auch diese Validierung durchlaufen müssen, bevor abgeschätzt werden kann, ob sie einsatzfähig sind. Zudem stellte die lange Versuchsdauer eine lange Wartezeit bis zur Freigabe von Produktionsreihen dar.

Bredäcs M. et al., Accelerated aging of polyethylene pipe grades in aqueous chlorine dioxide at constant concentration, Polymer Degradation and Stability, Bd. 157, November 2018, Seiten 80 bis 89, untersucht die Auswirkung von desinfiziertem Wasser auf den Abbau von Polyethylen (PE) durch Eintauchversuche von zwei PE-Rohrmaterialien in Lösungen bei unterschiedlichen Temperaturen, die mittels Natriumhypochlorid gebildetes Chlordioxid (ClO₂) enthalten.

Roy P.K. et al., Accelerated aging of LDPE films containing cobalt complexes as prooxidants, Polymer Degradation and Stability, Bd. 91, Nr. 8, August 2006, Seiten 1791 bis 1799, beschreibt die Synthese und Charakterisierung von zwei Kobaltkomplexen, nämlich Kobalt-Styrol-Maleat-Copolymer (CSMA) und Kobaltstearat (CS), und deren Auswirkung auf das Abbauverhalten von Polyethylen niedriger Dichte (LDPE).

Francesco Recupero et al., Free Radical Functionalization of Organic Compounds Catalyzed by N-Hydroxyphthalimide; Chemical Reviews, Bd. 107, Nr. 9, September 2007, Seiten 3800 bis 3842, untersucht Reaktionen unterschiedlicher niedermolekularer Substrate unter aerober Oxidation bei Einwirkung von N-Hydroxyphthalimid und Cobalt als Oxidationssystem für die Synthesechemie.

Die Aufgabe der Erfindung bestand somit in der Bereitstellung eines neuen beschleunigten Alterungsverfahrens, um die Entwicklungszyklen und die Sicherheit der Aussagen zur Alterung für Kunststoffartikel, insbesondere der fertigen Produkte, zu verbessern.

Die Erfinder haben festgestellt, dass dies durch Einsatz einer flüssigen Mischung zur Alterungsbehandlung von Kunststoffartikel erreicht werden kann, wobei die flüssige Mischung mindestens einen Oxidationskatalysator oder mindestens ein Oxidationsmittel und eine Säure sowie gegebenenfalls mindestens einen Cokatalysator für den Oxidationskatalysator umfasst.

Die Aufgabe wurde somit durch ein Verfahren zur beschleunigten Alterung eines Kunststoffartikels gelöst, wobei das Verfahren das Inkontaktbringen des Kunststoffartikels mit einer flüssigen Mischung umfasst, wobei die flüssige Mischung
a) mindestens einen Oxidationskatalysator und
b) gegebenenfalls mindestens einen Cokatalysator für den mindestens einen Oxidationskatalysator und
c) mindestens eine Säure und
d) gegebenenfalls Wasser und/oder mindestens ein anderes Lösungsmittel umfasst, wobei der mindestens eine Oxidationskatalysator ein N-Hydroxyimid ist.

In einer bevorzugten Ausführungsform umfasst die flüssige Mischung:
a) mindestens einen Oxidationskatalysator ausgewählt aus einem N-Hydroxyimid, insbesondere einem cyclischen N-Hydroxyimid, bevorzugt N-Hydroxyphthalimid und/oder N-Hydroxysuccinimid, und
b) mindestens einen Cokatalysator ausgewählt aus einem Übergangsmetallsalz oder einem Übergangsmetallkomplex und
c) mindestens eine Säure ausgewählt aus einer anorganischen Säure oder einer organischen Säure und
d) gegebenenfalls Wasser und/oder mindestens ein anderes Lösungsmittel.

Mit dem erfindungsgemäßen Alterungsverfahren ist es möglich, Kunststoffartikel in sehr kurzer Zeit, z.B. innerhalb von 1 Woche oder sogar innerhalt von 50 Stunden, in den selben Alterungszustand zu überführen, der bei üblichen Prüfungen unter Einsatzbedingungen erst nach 3000 Stunden gemäß den üblichen Protokollen erreicht wird. Dies stellt einen enormen Zeitgewinn dar, was allgemein die Entwicklungszeiten oder die Dauer von Materialprüfungen deutlich reduzieren kann.

Es hat sich auch vorteilhafterweise gezeigt, dass die Reproduzierbarkeit der Ergebnisse gewährleistet werden kann.

Das Verfahren, das in dieser Erfindung beschrieben ist, kann für Kunststoffe jeder Art eingesetzt werden. Die beschleunigte Alterung basiert dabei insbesondere auf dem Prinzip der thermisch oxidativen Alterung, welche durch die Säure zusätzlich beschleunigt wird. Dies kann auch ein Angriff der in Kunststoffen enthaltenen Alterungsinhibitoren und deren Abbau beinhalten. Alterungsinhibitoren können die Alterungseigenschaften der Kunststoffe wesentlich mitbestimmen, insbesondere bei Polyamiden. Auch Hydrolysereaktionen können bei der Alterung eine Rolle spielen.

Der Einsatz der erfindungsgemäß eingesetzten flüssigen Mischungen ist in diversen Gebieten denkbar. Kunststoffe altern und das muss bei der Konstruktion berücksichtigt werden. Daher müssen erste Abschätzungen schnellstmöglich vorliegen, um das Design des Produktes weiter zu verfeinern. Somit erstreckt sich die Anwendung auf alle Bereiche, in dem Kunststoffe einer Alterung unterliegen und diese im Vorfeld validiert werden müssen.

In den beigefügten Zeichnungen zeigt:
- Fig. 1: die relative Abnahme des E-Moduls des Kunststoffartikels während der Alterungsbehandlung gemäß Beispiel 1
- Fig. 2: die relative Abnahme der Bruchspannung des Kunststoffartikels während der Alterungsbehandlung gemäß Beispiel 1
- Fig. 3: die relative Abnahme der Bruchdehnung des Kunststoffartikels während der Alterungsbehandlung gemäß Beispiel 1
- Fig. 4: die Änderung des Carbonyl-Indexes des Kunststoffartikels in Abhängigkeit von der Behandlungsdauer während der Alterungsbehandlung gemäß Beispiel 1
- Fig. 5: die relative Abnahme der Bruchspannung des Kunststoffartikels während der Alterungsbehandlung gemäß der Beispiele 2 und 3.

Die Erfindung wird im Folgenden ausführlich beschrieben.

Unter dem Begriff der Alterung werden nach DIN 50035 alle irreversibel ablaufenden physikalischen und chemischen Änderungen der Werkstoffeigenschaften im Laufe der Zeit bezeichnet. Diese Veränderungen können unterschiedliche Ursachen haben und auf spezifische Abbaumechanismen zurückzuführen sein. In DIN 50035 werden die alternden Prozesse in chemische und physikalische Alterungsvorgänge unterteilt.

Eine beschleunigte Alterung kann z.B. eine Alterung im Vergleich zur Alterung bei Lagerung unter Standardklima gemäß DIN ISO 291 darstellen. Unter einer beschleunigten Alterung wird hier aber insbesondere eine beschleunigte Alterung im Vergleich zu Langzeitalterungen nach dem Stand der Technik verstanden, bei denen der Kunststoffartikel Bedingungen unterworfen wird, die den Bedingungen entsprechen, denen der Kunststoffartikel beim beabsichtigten Einsatz ausgesetzt ist. Ein solche Langzeitalterung erfolgt in der Regel in Testständen, in denen die Einsatzbedingungen für den Kunststoffartikel simuliert werden, gewöhnlich anhand von Spezifikationen des Kunden.

Der Alterungsgrad des Kunststoffartikels kann anhand unterschiedlicher Kennwerte bestimmt werden, z.B. aus Zugversuchen ermittelte mechanische Kennwerte, die u.a. von der Kettenlänge der Kunststoffpolymere abhängen, oder dem Anteil an enthaltenen Abbauprodukten, die durch IR-Spektroskopie bestimmt werden können.

Die flüssige Mischung umfasst mindestens einen Oxidationskatalysator.

Der mindestens eine Oxidationskatalysator ist ein N-Hydroxyimid, bevorzugt ein cyclisches N-Hydroxyimid. Bei einem cyclischen N-Hydroxyimid ist die N-Hydroxyimid-Struktur in einer Ringstruktur enthalten, vorzugsweise Succinimid, das gegebenenfalls Substituenten enthalten kann.

Besonders bevorzugt ist der mindestens eine Oxidationskatalysator N-Hydroxyphthalimid und/oder N-Hydroxysuccinimid, bevorzugt N-Hydroxyphthalimid oder N-Hydroxysuccinimid, wobei N-Hydroxyphthalimid besonders bevorzugt ist. N-Hydroxyphthalimid wird im Folgenden wie üblich mit NHPI und N-Hydroxysuccinimid mit NHS abgekürzt. NHPI zeigte eine kürzere Induktionsperiode im Vergleich zu NHS beim Umsatz von Substraten und im Allgemeine eine stärkere Beschleunigung der Alterung. NHS zeigte eine bessere Löslichkeit in hydrophilen Lösungen schon bei niedrigen Temperaturen im Vergleich zu NHPI.

Der Einsatz von N-Hydroxyimid-Derivaten als Katalysatoren für Oxidationsreaktionen stellen eine ungiftige und umweltverträgliche Alternative zu Katalysatoren aus toxischen Übergangsmetallen dar. Die N-Hydroxyimid-Derivate bilden nach Wasserstoffabstraktion von der -NOH-Gruppe durch Sauerstoff, das reaktive N-Oxy-Radikal, z.B. ein Phthalimid-N-oxy-Radikal (PINO), welches Oxidationsreaktionen katalysieren kann.

In einer besonders bevorzugten Ausführungsform umfasst die flüssige Mischung:
a) mindestens einen Oxidationskatalysator ausgewählt aus einem N-Hydroxyimid, insbesondere einem cyclischen N-Hydroxyimid, bevorzugt N-Hydroxyphthalimid und/oder N-Hydroxysuccinimid, und
b) mindestens einen Cokatalysator ausgewählt aus einem Übergangsmetallsalz oder einem Übergangsmetallkomplex und
c) mindestens eine Säure ausgewählt aus einer anorganischen Säure oder einer organischen Säure und
d) gegebenenfalls Wasser und/oder mindestens ein anderes Lösungsmittel.

Alle hier enthaltenen Angaben, einschließlich der Angaben zu den Einzelheiten zur Durchführung des Verfahrens, beziehen sich, sofern anwendbar, insbesondere auch auf diese besonders bevorzugte Ausführungsform, sofern nicht anders angegeben.

Die flüssige Mischung umfasst mindestens eine Säure. Es kann eine anorganische Säure und/oder eine organische Säure sein, wobei mindestens eine organische Säure bevorzugt ist. Ein bevorzugtes Beispiel für eine anorganische Säure ist Schwefelsäure. Ein bevorzugtes Beispiel für eine organische Säure ist mindestens eine C₁₋₄-Carbonsäure. Solche kurzkettigen Carbonsäuren sind bevorzugt, da sie besser in das Kunststoffmaterial eindringen können.

Die mindestens eine Säure ist bevorzugt ausgewählt aus Ameisensäure, Essigsäure, Propionsäure oder einer Mischung davon, wobei Ameisensäure und/oder Essigsäure besonders bevorzugt sind. Bei Einsatz von der besonders bevorzugten Kombination von Ameisensäure und Essigsäure kann das Volumenverhältnis von Essigsäure zu Ameisensäure z.B. im Bereich von 2:1 bis 20:1, bevorzugt 3:1 bis 15:1, liegen.

Die Säuren, insbesondere die Carbonsäuren, dienen insbesondere als Lösemittel für den Oxidationskatalysator und gegebenenfalls den Cokatalysator bzw. für das Oxidationsmittel. Darüber hinaus können die Säure katalytische für Hydrolysereaktionen oder andere Reaktionen aufweisen. Eine besonders geeignete Säure bei Einsatz von Natriumhypochlorit als Oxidationsmittel ist Schwefelsäure, aber auch der Einsatz der genannten organischen Säuren ist denkbar.

Die flüssige Mischung umfasst gegebenenfalls mindestens einen Cokatalysator. Die flüssige Mischung enthält bevorzugt mindestens einen Cokatalysator.

Der mindestens eine Cokatalysator ist bevorzugt ein Übergangsmetallsalz oder ein Übergangsmetallkomplex. Geeignete Beispiele für Übergangsmetallsalz oder Übergangsmetallkomplex sind solche, bei denen das Übergangsmetall des Übergangsmetallsalzes oder Übergangsmetallkomplexes aus Cu, z.B. Cu(II), Mn, z.B. Mn(II) oder Mn(III), Co, z.B. Co(II) oder Co(III), oder Fe ausgewählt ist. Besonders bevorzugt ist der Cokatalysator ein Cobaltsalz oder ein Cobaltkomplex, insbesondere ein Cobalt(II)-salz oder ein Cobalt(II)-komplex.

Beispiele für geeignete Übergangsmetallsalze bzw. -komplexe sind Halogenide, z.B. Chloride, Sulfate oder Acetate von Cu, Mn, Co oder Fe. Besonders bevorzugt ist Cobalt(II)-acetat (Co(Ac)₂).

Die Cokatalysatoren, insbesondere die Übergangsmetallsalze bzw. Übergangsmetallkomplex, unterstützen die oxidierende Wirkung des Oxidationskatalysators. Beispielweise generieren sie intermediäre Hydroperoxide, was die N-Oxy-Radikalbildung bei den genannten N-Hydroxyimid-Derivaten fördert.

Die Cokatalysatoren können auch Alterungsinhibitoren der Kunststoffe angreifen, wie z.B. Alterungsinhibitoren, die in Polyamiden enthalten sind.

Die flüssige Mischung kann ferner Wasser und/oder ein anderes Lösungsmittel enthalten. Ein Beispiel für ein von Wasser verschiedenes Lösungsmittel sind Alkohole, z.B. Ethanol. Es ist bevorzugt, dass die flüssige Mischung Wasser umfasst, d.h. die flüssige Mischung ist bevorzugt eine wässrige flüssige Mischung.

Es ist bevorzugt, dass der mindestens einen Oxidationskatalysator ausgewählt aus einem N-Hydroxyimid, und, falls vorhanden, der mindestens einen Cokatalysator, insbesondere das Übergangsmetallsalz oder der Übergangsmetallkomplex, in der flüssigen Mischung in gelöster Form vorliegen. Dies setzt nicht zwingend voraus, dass Oxidationskatalysator und gegebenenfalls Cokatalysator jeweils ganz gelöst sind, was aber vorteilhaft ist. Im Allgemeinen sollten z.B. jeweils mindestens 80 bis 100%, bevorzugt mindestens 90 bis 100%, von Oxidationskatalysator und gegebenenfalls Cokatalysator, zweckmäßigerweise nahezu 100%, in Lösung vorliegen. In diesem Sinne ist die flüssige Mischung bevorzugt eine Lösung. Nicht gelöste Bestandteile können gegebenenfalls als Flocken oder Partikel in der Lösung enthalten sein, was aber wie gesagt soweit wie möglich verhindert werden sollte. Dabei ist zu berücksichtigen, dass sich diese Angaben zur Lösung auf die flüssige Mischung beim Einsatz im erfindungsgemäßen Verfahren beziehen, bei dem die flüssige Mischung bevorzugt eine erhöhte Temperatur aufweist. Bekanntermaßen steigt die Löslichkeit von Verbindungen üblicherweise mit steigender Temperatur.

Die flüssige Mischung, bevorzugt die wässrige flüssige Mischung, enthält bevorzugt 0,03 bis 0,75 mol/L, bevorzugt 0,08 bis 0,4 mol/L, mindestens eines Oxidationskatalysators ausgewählt aus einem N-Hydroxyimid, insbesondere einem cyclischen N-Hydroxyimid, bevorzugt N-Hydroxyphthalimid und/oder N-Hydroxysuccinimid. Es versteht sich, dass sich diese und nachstehende Konzentrationsangaben auf mol Oxidationskatalysator bzw. Cokatalysator pro Liter flüssige Mischung bezieht.

Die flüssige Mischung, bevorzugt die wässrige flüssige Mischung, enthält bevorzugt 0,001 bis 0,1 mol/L, bevorzugt 0,005 bis 0,03 mol/L, mindestens eines Cokatalysators ausgewählt aus einem Übergangsmetallsalz oder einem Übergangsmetallkomplex, wobei das Übergangsmetall bevorzugt ausgewählt ist aus Cu, Mn, Co oder Fe, bevorzugter Co, insbesondere Co(II).

Die flüssige Mischung, bevorzugt die wässrige flüssige Mischung, enthält bevorzugt 35 bis 80 Vol.-%, bevorzugt 45 bis 70 Vol.-%, mindestens einer Säure ausgewählt aus einer C₁₋₄-Carbonsäure, bevorzugt Ameisensäure und/oder Essigsäure.

In einer bevorzugten Ausführungsform enthält die flüssige Mischung, bevorzugt die wässrige flüssige Mischung,
a) 0,03 bis 0,75 mol/L, bevorzugt 0,08 bis 0,4 mol/L, mindestens eines Oxidationskatalysators ausgewählt aus einem N-Hydroxyimid, insbesondere einem cyclischen N-Hydroxyimid, bevorzugt N-Hydroxyphthalimid und/oder N-Hydroxysuccinimid, und
b) 0,001 bis 0,1 mol/L, bevorzugt 0,005 bis 0,03 mol/L, mindestens eines Cokatalysators ausgewählt aus einem Übergangsmetallsalz oder einem Übergangsmetallkomplex, wobei das Übergangsmetall bevorzugt ausgewählt ist aus Cu, Mn, Co oder Fe, bevorzugter Co, insbesondere Co(II), und
c) 35 bis 80 Vol.-%, bevorzugt 45 bis 70 Vol.-%, mindestens einer Säure ausgewählt aus einer C₁₋₄-Carbonsäure, bevorzugt Ameisensäure und/oder Essigsäure.

Der zu behandelnde Kunststoffartikel kann z.B. der Kunststoff an sich, z.B. in Form eines Probeköpers, ein Halbzeug aus Kunststoff oder ein Bauteil bzw. ein Produkt, das zumindest eine Komponente aus dem Kunststoff aufweist, sein. Das Bauteil oder Produkt kann aus dem Kunststoff allein oder aus einer Kunststoffkomponente im Verbund mit einer oder mehreren Komponenten aus anderem Material gebildet sein, wobei ein Bauteil oder Produkt aus dem Kunststoff allein bevorzugt ist. Das erfindungsgemäße Verfahren ist insbesondere für Bauteile oder Produkte geeignet. Beispiele für zu prüfende Kunststoffartikel sind Kunststoffkupplungen, Getriebebauteile, Anlaufscheiben, Bauteile für Magnetspulen, Fahrzeugtanks sowie Schläuche, Hüllen, Verbindungselemente, z.B. für ein Tanksystem oder ein Kühlkreislaufsystem.

Es versteht sich, dass der Kunststoff ein oder mehrere Additive enthalten kann, wie sie auf dem Gebiet üblich sind. Beispiele für Additive sind Alterungsinhibitoren bzw. Stabilisatoren, Weichmacher, Pigmente, Verstärkungsmittel, z.B. Fasern oder teilchenförmige Partikel, z.B. Glasfasern oder mineralische Füllstoffe, Flammschutzmittel usw. Es kann sich z.B. um einen glasfaserverstärkten Kunststoff, z.B. ein glasfaserverstärktes Polyamid, handeln.

Der Kunststoff des Kunststoffartikels kann jeder beliebige Kunststoff sein, dessen Alterungsverhalten geprüft werden soll. Der Kunststoff des Kunststoffartikels ist bevorzugt ein durch Polyadditions- oder Polykondensationsreaktion erhaltener Kunststoff, d.h. ein Polykondensat-Kunststoff oder ein Polyaddukt-Kunststoff. Der Kunststoff des Kunststoffartikels ist bevorzugt ein Polyester oder ein Polyamid, wobei Polyamid besonders bevorzugt ist. Beispiele für Polyamide (PA) sind PA 6.6, PA 4.6, PA 6, PA6.12 und PA 11.

Bei der Alterungsbehandlung des erfindungsgemäßen Verfahrens wird der Kunststoffartikel mit der flüssigen Mischung in Kontakt gebracht. Um den Kunststoffartikel mit der flüssigen Mischung in Kontakt zu bringen, kann der Kunststoffartikel in die flüssige Mischung getaucht werden, der Kunststoffartikel kann mit der flüssigen Mischung gespült oder durchspült werden oder der Kunststoffartikel kann mit der flüssigen Mischung befüllt werden. Beim Spülen bzw. Durchspülen oder Befüllen mit der flüssigen Mischung ist es möglich, nur eine Seite des Kunststoffartikels, z.B. die Innenseite, mit der flüssigen Mischung in Kontakt zu bringen. Ein Spülen bzw. Durchspülen oder Befüllen ermöglicht somit eine einseitige Belastung des Artikels, wodurch eine Belastung erreicht werden kann, die ähnlicher zum Praxisbetrieb sein kann.

Eine Befüllung des Kunststoffartikels hängt von der Geometrie des Artikels ab und kann gegebenenfalls ein Verschließen des Artikels nach der Befüllung einschließen. In der Regel weist der Kunststoffartikel bei einer Befüllung mindestens einen Hohlkörper auf. Gegebenenfalls kann auch das Verschließen einer Seite des Artikels vor dem Befüllen erforderlich sein, z.B. bei einem Schlauch.

Bei der Alterungsbehandlung des erfindungsgemäßen Verfahrens wird in einer Ausführungsform der Kunststoffartikel in die flüssige Mischung getaucht. Der Kunststoffartikel kann gegebenenfalls nur teilweise in die flüssige Mischung getaucht werden, in der Regel ist es aber zweckmäßiger, dass der gesamte Kunststoffartikel in die flüssige Mischung getaucht wird.

Zum Tauchen wird der Kunststoffartikel zweckmäßigerweise z.B. in einen geeigneten Behälter oder Kolben gelegt oder darin mit entsprechenden Fixierelementen fixiert und die flüssige Mischung eingefüllt. Natürlich kann die Reihenfolge auch umgedreht werden. Es ist vorteilhaft, wenn die flüssige Mischung während der Alterungsbehandlung in Bewegung gehalten wird, z.B. mithilfe eines Rührers. Wenn die Alterungsbehandlung bei erhöhter Temperatur, z.B. in der Nähe des Siedepunktes, und/oder unter Einleitung eines sauerstoffhaltigen Gases durchgeführt wird, ist es häufig zweckmäßig, den Behälter bzw. Kolben mit einem Rückflusskühler zu versehen, um ein Verdampfen von Flüssigkeit zu verhindern. Es versteht sich, dass für die Durchführung bei erhöhter Temperatur eine geeignete Heizvorrichtung vorzusehen ist.

Die Alterungsbehandlung bzw. das Inkontaktbringen des Kunststoffartikels mit der flüssigen Mischung, z.B durch Tauchen, Spülen bzw. Durchspülen oder Befüllen in bzw. mit der flüssigen Mischung, kann z.B. bei einer Temperatur im Bereich von 15°C bis 100°C erfolgen. Möglich ist auch eine Alterungsbehandlung bei einer Temperatur über dem Siedepunkt der flüssigen Mischung bzw. der darin enthaltenen Komponenten, dies bedingt aber eine Behandlung unter Druck, wofür eine aufwendigere Apparatur, z.B. ein Druckbehälter, benötigt wird. Dies ist nicht bevorzugt, da das Verfahren möglichst einfach gehalten werden soll.

Die Alterungsbehandlung bzw. das Inkontaktbringen des Kunststoffartikels mit der flüssigen Mischung, z.B. durch Tauchen, Spülen bzw. Durchspülen oder Befüllen in bzw. mit der flüssigen Mischung, wird bevorzugt bei einer Temperatur im Bereich von 60°C bis 90°C, bevorzugter 70 bis 85°C, durchgeführt. Eine Temperatur von ca. 80°C hat sich in der Praxis als besonders günstig erwiesen.

In einer bevorzugten Ausführungsform kann während der Alterungsbehandlung ein sauerstoffhaltiges Gas, insbesondere Luft, in die flüssige Mischung eingeleitet werden. Dies kann einfach mithilfe eines in die flüssige Mischung tauchenden Rohrs oder Schlauchs bewerkstelligt werden, welches bzw. welcher mit einer Pumpe und/oder einer Quelle für das sauerstoffhaltige Gas verbunden ist.

Die Dauer der Alterungsbehandlung kann von mehreren Faktoren abhängen, z.B. Zusammensetzung der flüssigen Mischung bzw. Konzentrationen der enthaltenen Komponenten, zu erreichender Alterungsgrad oder eingesetzte Temperatur. Die Alterungsbehandlung bzw. das Inkontaktbringen des Kunststoffartikels in die flüssige Mischung, z.B. durch Tauchen, Spülen, Durchspülen oder Befüllen in bzw. mit der flüssigen Mischung, kann z.B. über einen Zeitraum von 6 Stunden bis 300 Stunden, bevorzugt 20 Stunden bis 80 Stunden, erfolgen. Eine Dauer von ca. 50 Stunden hat sich in der Praxis als besonders zweckmäßig erwiesen. Sofern die Alterungsbehandlung bei einer erhöhten Temperatur, wie den vorstehend genannten Temperaturen, durchgeführt wird, ist es gegebenenfalls möglich, die Alterungsbehandlung zeitweise zu unterbrechen, indem die flüssige Mischung nicht mehr erwärmt wird, so dass sie auf Raumtemperatur abkühlt. Die Zeiten ohne Erwärmung werden für die Dauer der Alterungsbehandlung dann nicht berücksichtigt.

Nach der Alterungsbehandlung wird der Kunststoffartikel aus der flüssigen Mischung entnommen und gegebenenfalls anhaftende Reste der flüssigen Mischung durch Spülen mit und/oder Lagerung in einem Spülmedium, wie z.B. Wasser oder Deionat, entfernt. Der Praktiker kann durch visuelle Prüfung des erhaltenen Kunststoffartikels bereits Anhaltspunkte zum erreichten Alterungsgrad erhalten, z.B. anhand von Rissbildungen oder Verfärbungen. In der Regel werden aber für eine genauere Untersuchung des Alterungsgrades Analyseverfahren zur Bestimmung mindestens einer charakteristischen Eigenschaft des Kunststoffartikels eingesetzt.

Vor Durchführung solcher Analyseverfahren kann es zweckmäßig sein, den der Alterungsbehandlung unterworfenen Kunststoffartikel einer Rückkonditionierungsbehandlung zu unterziehen. Hierbei handelt es sich um eine Konditionierungsbehandlung, um den Kunststoffartikel bezüglich bestimmter Parameter, z.B. des Wassergehalts, wieder in den Zustand vor der Alterungsbehandlung zu bringen. Beispielsweise sind bestimmte Kunststoffe, wie z.B. Polyamide, in der Lage, Wasser bzw. Feuchtigkeit aus der Umgebung aufzunehmen, so dass sich der Wassergehalt während der Alterungsbehandlung erhöhen kann. Der Wassergehalt kann aber die Ergebnisse von Analyseverfahren, insbesondere von mechanischen Prüfungen, beeinflussen. Zur Vergleichbarkeit der Ergebnisse mit unbehandelten Proben ist daher eine Rückkonditionierung zur Standardisierung häufig zweckmäßig bzw. notwendig.

Zur Rückkonditionierung bzw. Konditionierung kann der Kunststoffartikel einem Konditionierklima unterworfen werden, bis sich ein Gleichgewicht zwischen der Temperatur und Feuchtigkeit des Artikels und dem Konditionierklima einstellt. Die Durchführung der Konditionierung kann z.B. nach dem Standardklima gemäß DIN EN ISO 291 (23°C, relative Luftfeuchtigkeit von 55%) erfolgen.

Das erfindungsgemäße Verfahren umfasst daher in einer bevorzugten Ausführungsform ferner das Analysieren des Kunststoffartikels nach Entnahme aus der flüssigen Mischung und gegebenenfalls einer Konditionierungsbehandlung, um mindestens eine Eigenschaft des der Alterungsbehandlung unterworfenen Kunststoffartikels zu bestimmen. Es versteht sich, dass die zu bestimmenden Eigenschaften insbesondere Eigenschaften sind, die für den Alterungsgrad charakteristisch sind. Geeignete Analyseverfahren zur Bestimmung des Alterungsgrades von Kunststoffartikeln sind z.B. in DE102018214235A1, DE102016213188A1 und DE102016213157A1 beschrieben, auf die hier Bezug genommen wird.

Beispiele für weitere geeignete Analyseverfahren sind mechanische Prüfungen zur Ermittlung mechanischer Kennwerte, z.B. mechanische Zugprüfung, dynamische Differenzkalorimetrie, IR-Spektroskopie und optische Analyseverfahren, wie z.B. Lichtmikroskopie und Rasterelektronenmikroskopie (REM) Es können eine oder mehrere Analyseverfahren eingesetzt werden. In einer bevorzugten Ausführungsform umfasst die Analyse des Kunststoffartikels mindestens ein Analyseverfahren ausgewählt aus mechanischer Zugprüfung, dynamischer Differenzkalorimetrie, optischer Analyse und IR-Spektroskopie, bevorzugter mechanischer Zugprüfung und/oder IR-Spektroskopie.

Die IR-Spektroskopie, z.B. FTIR-Spektroskopie oder FTIR-ATR-Spektroskopie, eignet sich zur Ermittlung von Abbauprodukten. Die mechanischen Kennwerte aus den Zugversuchen sind u.a. ein Indiz für die Länge der Polymerketten. Aus der Differenzkalorimetrie ergeben sich Erkenntnisse zu den kristallinen Phasen im Kunststoff.

Die Erfindung betrifft auch die Verwendung einer flüssigen Mischung als Alterungsmedium zur beschleunigten Alterung eines Kunststoffartikels, wobei die flüssige Mischung
a) mindestens einen Oxidationskatalysator und
b) gegebenenfalls mindestens einen Cokatalysator für den mindestens einen Oxidationskatalysator und
c) mindestens eine Säure und
d) gegebenenfalls Wasser und/oder mindestens ein anderes Lösungsmittel umfasst, wobei der mindestens eine Oxidationskatalysator ein N-Hydroxyimid ist.

Es versteht sich, dass für die erfindungsgemäße Verwendung die vorstehenden Angaben für das Verfahren zur flüssigen Mischung, zum Kunststoffartikel und zum Verfahren in gleicher Weise gelten, so dass darauf verwiesen wird.

Die Erfindung betrifft auch eine flüssige Mischung, bevorzugt eine wässrige flüssige Mischung, zur beschleunigten Alterung eines Kunststoffartikels, umfassend
a) 0,03 bis 0,75 mol/L, bevorzugt 0,08 bis 0,4 mol/L, mindestens eines Oxidationskatalysators ausgewählt aus einem N-Hydroxyimid, insbesondere einem cyclischen N-Hydroxyimid, bevorzugt N-Hydroxyphthalimid und/oder N-Hydroxysuccinimid, und
b) 0,001 bis 0,1 mol/L, bevorzugt 0,005 bis 0,03 mol/L, mindestens eines Cokatalysators ausgewählt aus einem Übergangsmetallsalz oder einem Übergangsmetallkomplex, wobei das Übergangsmetall bevorzugt ausgewählt ist aus Cu, Mn, Co oder Fe, bevorzugter Co, und
c) 35 bis 80 Vol.-%, bevorzugt 45 bis 70 Vol.-%, mindestens einer Säure ausgewählt aus einer C₁₋₄-Carbonsäure, bevorzugt Ameisensäure und/oder Essigsäure.

Es versteht sich, dass für die erfindungsgemäße flüssige Mischung die vorstehenden Angaben zur flüssigen Mischung soweit anwendbar in gleicher Weise gelten, so dass darauf verwiesen wird.

Die Erfindung wird nun anhand von Ausführungsbeispielen weiter erläutert. Die konkreten Ausführungsbeispiele sollen die Erfindung in keiner Weise einschränken.

### Beispiele

Aufgrund einzuhaltender Sicherheitsvorschriften konnten die Versuche mit erhöhter Temperatur nur tagsüber betrieben werden. Es wird aber davon ausgegangen, dass die Stillstandzeiten bei Raumtemperatur über Nacht keinen wesentlichen Einfluss auf die Versuchsergebnisse haben und daher vernachlässigbar sind. Es bietet sich an, den Betrieb ohne Unterbrechung über Nacht durchzuführen, um die Alterung weiter zu beschleunigen.

### Zugversuch

Mittels Zugprüfung können Kennwerte wie E-Modul, Bruchdehnung und Bruchspannung ermittelt werden. Für die Messung wurde eine Material-Prüfmaschine des Typs Z010 der Firma Zwick verwendet. Es wurde eine Dreifachbestimmung gemäß Prüfnorm DIN EN ISO 527-2. 2012-06 mit einer Zuggeschwindigkeit von 50 mm/s durchgeführt. Da sich die Dimensionen der Schulterstäbe nach der Alterung leicht verändern können, wurden diesbezüglich Korrekturen vor der Messung vorgenommen. Vor der Messung wurden die gealterten Proben einer Rückkonditionierung unterworfen, wie nachstehend beschrieben.

### FTIR-ATR-Spektroskopie

Die Schulterstäbe wurden zunächst aus dem Wasserbad entnommen und für 24 Stunden an der Luft vorgetrocknet. Anschließend erfolgte eine Endtrocknung bei 80 °C für 12 Stunden im Trockenschrank. Für die Messung an Rohrkupplungen wurden mittels einer Säge Probenstücke herausgetrennt, die ebenfalls bei 80 °C im Trockenschrank getrocknet wurden.

Es wurde das FTIR-Spektrometer "Spectrum Two" der Firma Perkin Elmer eingesetzt. Als ATR-Kristall wurden Diamant für die Auswertung herangezogen. Der Wellenzahlenbereich von 4000 - 400 cm⁻¹ wurde in acht Zyklen pro Messung abgefahren (Dreifachmessung an unterschiedlichen Stellen des Schulterstabes).

Für die quantitative Auswertung wurde der Messbereich zwischen 1800 - 1700 cm⁻¹ genauer untersucht. In diesem Bereich finden sich typische Absorptions-banden der Alterungsprodukte. Als interner Standard diente die CH2-Bande mit einem Maximum bei ca. 2928 cm⁻¹. Die quantitative Auswertung erfolgt über Bildung des Verhältnisses der Peak-Gesamtfläche im Wellenzahlbereich von 1800 bis 1700 cm⁻¹ zur Peakfläche des internen Standards (CH2; ~2928 cm⁻¹). Im Folgenden wird dieses Verhältnis als "Carbonyl-Index" bezeichnet.

### Beispiel 1

Bei den untersuchten Proben handelt es sich um spritzgegossene Schulterstäbe entsprechend den Prüfnormen DIN EN ISO 527-2 aus glasfaserverstärktem und mit Ruß gefärbtem Polyamid 6.6.

Die als Alterungsmedium eingesetzte flüssige Mischung bzw. Lösung wies folgende Zusammensetzung auf:

| | |
|---|---|
| NHPI | 0,15 mol/L |
| Co(Ac)₂ | 0,0075 mol/L |
| Essigsäure | 50 Vol.-% |
| Ameisensäure | 5 Vol.-% |
| destilliertes Wasser | Rest |

Ein Druckbehälter der Firma Kliewe GmbH wurde mit 800 mL des Alterungsmediums befüllt. Die Prüfstäbe wurden lose in die Lösung eingelegt. Das Medium wurde mit einem PTFE-Rührfisch gerührt. Über einen Laborkompressor wurde während den Heizzeiten kontinuierlich mit einem Sterilfilter gereinigte Luft in die Lösung eingeleitet. Da durch das Einleiten von Luftsauerstoff mittels Kompressor sich hohe Dampfverluste ergeben können, wurde ein mittels Wasserbad gekühlter Kondensatsammler angebracht.

Das Alterungsmedium wurde für ca. 10 Stunden pro Tag auf 80 °C erhitzt und aktiv belüftet. Abgesehen von den relativ kurzen Aufheiz- und Abkühlphasen war lagerten die Prüfstäbe für die restliche Zeit bei Raumtemperatur (ca. 20°C) in dem Alterungsmedium. Die Alterungsbehandlung wurde über einen Zeitraum von 1 Woche durchgeführt. Für die Dauer der Alterungsbehandlung sind nur die Zeiten bei erhöhter Temperatur relevant, die Zeiten bei Raumtemperatur werden nicht berücksichtigt.

In regelmäßigen Abständen gemäß folgender Tabelle wurden Prüfstäbe aus dem Alterungsmedium entnommen, mit Deionat gespült und in einem Wasserbad gelagert, damit eindiffundierte Alterungsmedienrückstände aus der Polyamidmatrix entfernt werden.

| Entnahme Probenreihe | Dauer der aktiven Beheizung bei 80 °C [h] | Lagerungsdauer bei RT im Alterungsmedium [h] |
|---|---|---|
| N1-Reihe | 5,0 | 0 |
| N2-Reihe | 10,0 | 11,0 |
| N3-Reihe | 20,0 | 11,0 |
| N4-Reihe | 30,0 | 23,0 |
| N5-Reihe | 40,0 | 34,0 |
| N6-Reihe | 50,0 | 44,5 |

Für die Bestimmung der mechanischen Kennwerte wurden die Proben aus dem Wasserbad entnommen und zunächst an der Luft für ca. acht Stunden vorgetrocknet. Da die mechanischen Kennwerte stark mit dem Wassergehalt in der Polyamid-Matrix variieren, wurden die Prüfstäbe anschließend für mindestens 72 Stunden im Exsikkator über einer übersättigten Magnesiumnitrat-Lösung bei Raumtemperatur (ca. 20 °C) und einer rel. Luftfeuchtigkeit zwischen 49,9 - 55,0 % rückkonditioniert.

Zum Vergleich wurden Kennwerte an nicht der Alterungsbehandlung unterworfenen Proben (= Nullprobe bzw. 0-Probe) bestimmt, die als 100% Wert genommen wurden. Die Bruchspannung der Nullprobe lag zwischen 107 - 109 MPa bei Bruchdehnungswerten von 4,5 - 5,9 %. Die Ergebnisse für die gealterten Proben sind im Folgenden dargestellt.

### Optische Analyse

Es konnten bereits makroskopisch schwerwiegende Schädigungen der Schulterstäbe in der NHPI/Co(Ac)2-Lösung in Abhängigkeit ihrer Behandlungszeit, festgestellt werden, was durch Analyse lichtmikroskopischer und REM-Aufnahmen bestätigt wurde.

### Zugprüfung

Die Ergebnisse der N6-Proben sind als Tendenzen anzusehen sind, da es durch Kerben im Bereich des Steges zu einer frühzeitigen Ermüdung des Materials kam.

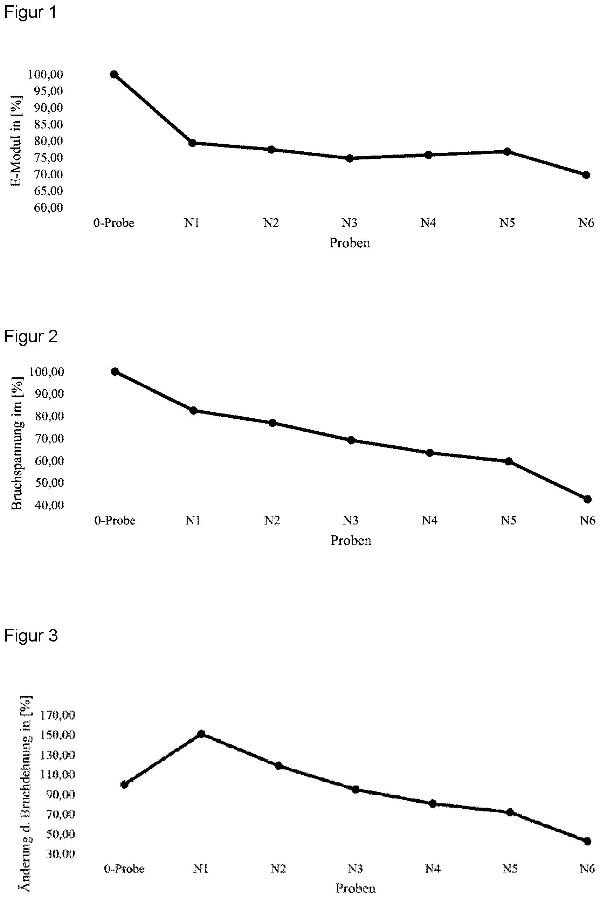

Fig. 1 zeigt die relative Abnahme des E-Moduls während der Alterungsbehandlung, aus dem ein deutlichen Abfall auf ca. 80 % nach den ersten fünf Stunden aktiver Beheizung ersichtlich wird.

Die in Fig. 2 gezeigte relative Bruchspannung zeigt ebenfalls einen steilen Abfall innerhalb der ersten fünf Stunden. Im Anschluss ist ein linear abnehmender Trend erkennbar. Bereits die fünfte Entnahmestelle zeigt eine Bruchspannung von lediglich 60 %.

Bei der in Fig. 3 gezeigten Bruchdehnung zeigt sich zunächst eine Verbesserung der Bruchdehnung in den ersten Stunden, die jedoch anschließend drastisch abnimmt. Bei der dritten Entnahmestelle ist eine Abnahme der Bruchdehnung auf ca. 95 % und bei der fünften Entnahmestelle eine Bruchdehnung von sogar nur noch 70 % des ursprünglichen Wertes zu beobachten.

Sowohl die Abnahme der Bruchspannung als auch der Bruchdehnung lassen auf einen stetigen Abbau der Polyamid-Matrix schließen.

### FTIR-ATR-Spektroskopie

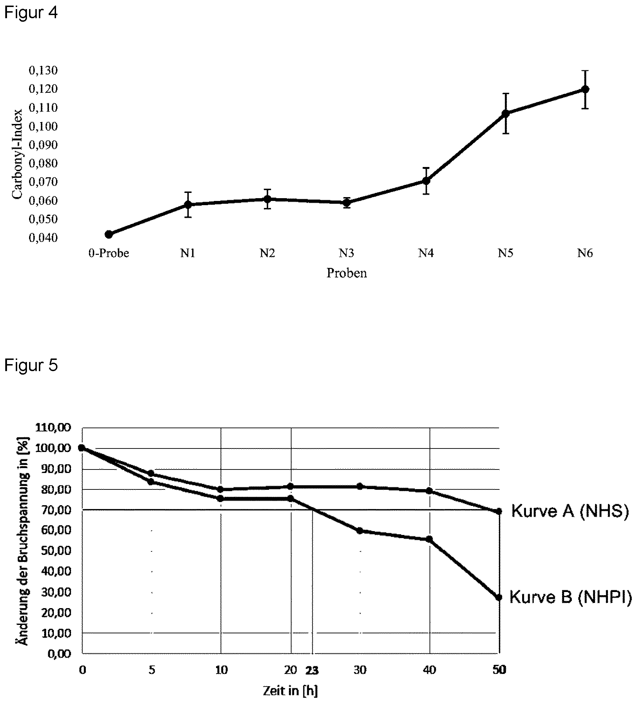

Die Änderung des vorstehend beschriebenen Carbonyl-Indexes in Abhängigkeit zur Behandlungsdauer ist in Fig. 4 dargestellt. Bei der Bildung des Carbonyl-Indexes zeigt sich ein deutlicher Anstieg mit zunehmender Behandlungsdauer. Die Ergebnisse bestätigen, dass neben der Hydrolyse der Imid-Bande auch eine voranschreitende Alterung der Matrix stattgefunden hat.

### Vergleich

Die in Beispiel 1 erhaltenen Ergebnisse wurden mit Messergebnissen für Schulterstäbe aus glasfaserverstärktem Polyamid 6.6 (für Zugprüfungen) und Rohrkupplungen (für FTIR-Messungen) verglichen, die in einem Gemisch aus Wasser und Kühlmittel unter vergleichbaren Bedingungen gealtert worden sind, wie sie in einem Teststand für einen Kühlmittelkreislauf nach dem Stand der Technik herrschen (Temperaturen von ca. 120°C und erhöhter Druck).

Insgesamt bewegen sich die Bruchspannungswerte des Beispiels 1 und der im Kühlmittel gealterten Proben im gleichen Größenbereich. Allerdings zeigen die gemäß Beispiel 1 gealterten Proben eine deutlich beschleunigte Alterung. So entspricht gemäß den ermittelten Werten für die Bruchspannung eine Behandlung von 30 h aktiver Beheizung in Beispiel 1 einer Behandlungsdauer von ca. 1750 h der im Kühlmittel gealterten Proben.

Auch ein Vergleich mit der zeitlichen Änderung der Bruchdehnung zeigt eine sehr gute Übereinstimmung der beiden Systeme, wenn auch in deutlich unterschiedlichen Zeitintervallen (Beispiel 1: 0-50 h (Zeiten bei erhöhter Temperatur); in Kühlmittel gealterte Proben: 0-3000 h).

Ein Vergleich des Carbonyl-Indexes der im Kühlmittel gealterten Rohrkupplung mit den Proben von Beispiel 1 zeigt, dass eine vergleichbare Menge an Abbauprodukten bei ca. 50 h aktiver Beheizung in Beispiel 1 erst nach 2.861 h in der im Kühlmittel gealterten Probe erreicht wird.

Auch die Analyse der durch Licht- und Rasterelektronenmikroskopie erhaltenen Aufnahmen zeigt eine gute Übereinstimmung zwischen den im Kühlmittelgemisch gealterten Proben und denen aus Beispiel 1.

Die Untersuchungen belegen, dass mit dem NHPI/Co(Ac)₂-System eine rapide Zeitraffung des Alterungsprozesses von Polyamid 6.6-GF30 möglich ist. Ein Vergleich mit Proben aus Alterungsversuchen in Kühlmittel/Deionat-Mischungen und Rohrkupplungen aus dem Teststand zeigen weitestgehend eine sehr gute Übereinstimmung bezüglich der Veränderung der mechanischen Kennwerte und Vergleichbarkeit bezüglich der Verteilung der Abbauprodukte.

### Beispiel 2

Bei den untersuchten Proben handelt es sich um spritzgegossene Schulterstäbe entsprechend den Prüfnormen DIN EN ISO 527-2 aus glasfaserverstärktem Polyamid 6.6. Der eingesetzte Kunststoff war von dem in Beispiel 1 eingesetzten Polyamidtyp verschieden (unterschiedliche Hersteller).

Die als Alterungsmedium eingesetzte flüssige Mischung bzw. Lösung wies folgende Zusammensetzung auf:

| | |
|---|---|
| NHS | 0,2 mol/L |
| Co(Ac)2 | 0,01 mol/L |
| Essigsäure | 50 Vol.-% |
| Ameisensäure | 5 Vol.-% |
| destilliertes Wasser | Rest |

Für die Versuche wurde eine Standardrückflussapparatur aus einem 2 L Glasrundkolben mit aufgesetztem Dimroth-Rückflusskühler und einer Heizhaube eingesetzt. Eine gleichmäßige Temperatur wurde durch thermische Konvektion erreicht. Die aktive Belüftung des Mediums mit Luftsauerstoff bei erhöhter Temperatur wurde über einen Laborkompressor ermöglicht. Die Proben wurden auf einem biegsamen Edelstahldraht aufgefädelt und an ein Edelstahlgitter befestigt, das in den Kolben platziert wurde. Der Kolben wurde dann mit der flüssigen Mischung gefüllt.

Die Parameter und die Art der Versuchsdurchführung, wie z.B. Temperaturprofil und Messverfahren, entsprachen ansonsten denen von Beispiel 1.

In Fig. 5 ist die Änderung der Bruchspannung im Verlauf der Alterungsbehandlung als Kurve A dargestellt. Zu beachten ist, dass auf der Zeitachse nur die Dauer der Behandlung bei 80°C berücksichtigt ist. D.h. die Stilstandzeiten bei Raumtemperatur sind nicht berücksichtigt.

Die ermittelten mechanischen Kennwerte bestätigen, dass das System auf Basis von NHS den Alterungsprozess beschleunigt. Sowohl eine Konzentrationserhöhung des Katalysators als auch eine Temperaturerhöhung konnte die zeitraffende Wirkung nur geringfügig verbessern.

### Beispiel 3

Zum Vergleich wurden die gleichen Versuche wie in Beispiel 2 durchgeführt, außer dass das Alterungsmedium durch das Alterungsmedium von Beispiel 1 ersetzt wurde. Die sich ergebende Änderung der Bruchspannung im Verlauf der Alterungsbehandlung ist ebenfalls in Fig. 5 gezeigt (Kurve B).

Der Vergleich mit dem System von Beispiel 2 auf Basis von NHS zeigt ab ca. 20 Stunden Behandlung eine deutlich schnellere Alterung beim NHPI-System gemäß Beispiel 3 gegenüber dem NHS-System von Beispiel 2.

### Beispiel 4

Anhand der erhaltenen Ergebnisse kann ein beschleunigtes Alterungsverfahren für Kunststoffbauteile wie eine Kunststoffkupplung aus PA66-GF30 (Quick Connector) konzipiert werden. Hier wird die Kupplung z.B. in einem Alterungsmedium gemäß Beispiel 1 eingetaucht. Die Lösung wird kontinuierlich durch einen Rührer in Bewegung gehalten. Die Alterung setzt durch die Einwirkung einer erhöhten Temperatur, z.B. 80°C, und der Lösung ein. Zweckmäßigerweise wird während der Behandlung Luft in die Lösung eingeleitet. Das Bauteil wird nach der Alterungsbehandlung (z.B. für 50 Stunden) aus dem Medium herausgenommen, gespült und rückkonditioniert. Diese Ergebnisse der Materialprüfung können an diesem Bauteil ermittelt werden.

## Patentansprüche

1. Verfahren zur beschleunigten Alterung eines Kunststoffartikels, umfassend das Inkontaktbringen des Kunststoffartikels mit einer flüssigen Mischung, umfassend
a) mindestens einen Oxidationskatalysator und
b) gegebenenfalls mindestens einen Cokatalysator für den mindestens einen Oxidationskatalysator und
c) mindestens eine Säure und
d) gegebenenfalls Wasser und/oder mindestens ein anderes Lösungsmittel, **dadurch gekennzeichnet, dass** der mindestens eine Oxidationskatalysator ein N-Hydroxyimid ist.

2. Verfahren nach Anspruch 1, wobei der mindestens eine Oxidationskatalysator ein cyclisches N-Hydroxyimid, bevorzugt N-Hydroxyphthalimid und/oder N-Hydroxysuccinimid, ist.

3. Verfahren nach einem der vorhergehenden Ansprüche, wobei die flüssige Mischung umfasst:
a) mindestens einen Oxidationskatalysator ausgewählt aus einem N-Hydroxyimid, insbesondere einem cyclischen N-Hydroxyimid, bevorzugt N-Hydroxyphthalimid und/oder N-Hydroxysuccinimid, und
b) mindestens einen Cokatalysator ausgewählt aus einem Übergangsmetallsalz oder einem Übergangsmetallkomplex und
c) mindestens eine Säure ausgewählt aus einer anorganischen Säure oder einer organischen Säure und
d) gegebenenfalls Wasser und/oder mindestens ein anderes Lösungsmittel.

4. Verfahren nach einem der vorhergehenden Ansprüche, wobei die Säure Schwefelsäure oder mindestens eine C₁₋₄-Carbonsäure ist.

5. Verfahren nach einem der vorhergehenden Ansprüche, wobei die Säure ausgewählt ist aus Ameisensäure, Essigsäure, Propionsäure oder einer Mischung davon, bevorzugt Ameisensäure und/oder Essigsäure.

6. Verfahren nach Anspruch 3, wobei das Übergangsmetall des Übergangsmetallsalzes oder Übergangsmetallkomplexes ausgewählt ist aus Cu, Mn, Co oder Fe, bevorzugt Co.

7. Verfahren nach einem der vorhergehenden Ansprüche,
wobei die flüssige Mischung Wasser umfasst, und/oder
wobei der mindestens einen Oxidationskatalysator ausgewählt aus einem N-Hydroxyimid, und, falls vorhanden, der mindestens einen Cokatalysator, insbesondere das Übergangsmetallsalz oder der Übergangsmetallkomplex, in der flüssigen Mischung in gelöster Form vorliegen.

8. Verfahren nach einem der vorhergehenden Ansprüche, wobei die flüssige Mischung, bevorzugt die wässrige flüssige Mischung,
a) 0,03 bis 0,75 mol/L, bevorzugt 0,08 bis 0,4 mol/L, mindestens eines Oxidationskatalysators ausgewählt aus einem N-Hydroxyimid, insbesondere einem cyclischen N-Hydroxyimid, bevorzugt N-Hydroxyphthalimid und/oder N-Hydroxysuccinimid, und
b) 0,001 bis 0,1 mol/L, bevorzugt 0,005 bis 0,03 mol/L, mindestens eines Cokatalysators ausgewählt aus einem Übergangsmetallsalz oder einem Übergangsmetallkomplex, wobei das Übergangsmetall bevorzugt ausgewählt ist aus Cu, Mn, Co oder Fe, bevorzugter Co, und
c) 35 bis 80 Vol.-%, bevorzugt 45 bis 70 Vol.-%, mindestens einer Säure ausgewählt aus einer C₁₋₄-Carbonsäure, bevorzugt Ameisensäure und/oder Essigsäure,
enthält.

9. Verfahren nach einem der vorhergehenden Ansprüche, wobei der Kunststoff des Kunststoffartikels ein Polykondensat-Kunststoff oder ein Polyaddukt-Kunststoff ist, wobei der Kunststoff bevorzugt ein Polyester oder ein Polyamid, bevorzugt ein Polyamid, ist.

10. Verfahren nach einem der vorhergehenden Ansprüche,
wobei das Inkontaktbringen des Kunststoffartikels mit der flüssigen Mischung bei einer Temperatur im Bereich von 15°C bis 100°C, bevorzugt im Bereich von 60°C bis 90°C, bevorzugter von 70 bis 85°C, erfolgt, und/oder,
wobei das Inkontaktbringen des Kunststoffartikels mit der flüssigen Mischung über einen Zeitraum von 6 Stunden bis 300 Stunden, bevorzugt 20 Stunden bis 80 Stunden, erfolgt.

11. Verfahren nach einem der vorhergehenden Ansprüche, wobei während der Alterungsbehandlung ein sauerstoffhaltiges Gas, insbesondere Luft, in die flüssige Mischung eingeleitet wird.

12. Verfahren nach einem der vorhergehenden Ansprüche, ferner umfassend das Analysieren des Kunststoffartikels nach seiner Entnahme aus der flüssigen Mischung zur Bestimmung mindestens einer Eigenschaft des der Alterungsbehandlung unterworfenen Kunststoffartikels, wobei der entnommene Kunststoffartikel vor der Analyse gegebenenfalls einer Rückkonditionierungsbehandlung unterworfen wird.

13. Verfahren nach Anspruch 12, wobei die Analyse des Kunststoffartikels mindestens eine von mechanischer Zugprüfung, dynamischer Differenzkalorimetrie, IR-Spektroskopie, optischer Analyse oder einer Kombination davon umfasst.

14. Verfahren nach einem der vorhergehenden Ansprüche, wobei der Kunststoffartikel mit der flüssigen Mischung in Kontakt gebracht wird, indem der Kunststoffartikel in die flüssige Mischung getaucht wird oder der Kunststoffartikel mit der flüssigen Mischung gespült oder durchspült wird oder der Kunststoffartikel mit der flüssigen Mischung befüllt wird.

15. Verwendung einer flüssigen Mischung als Alterungsmedium zur beschleunigten Alterung eines Kunststoffartikels, wobei die flüssige Mischung
a) mindestens einen Oxidationskatalysator und
b) gegebenenfalls mindestens einen Cokatalysator für den mindestens einen Oxidationskatalysator und
c) mindestens eine Säure und
d) gegebenenfalls Wasser und/oder mindestens ein anderes Lösungsmittel umfasst,
**dadurch gekennzeichnet, dass** der mindestens eine Oxidationskatalysator ein N-Hydroxyimid ist.

16. Verwendung nach Anspruch 15, wobei die flüssige Mischung wie in einem der Ansprüche 2 bis 8 definiert ist und/oder der Kunststoffartikel wie in Anspruch 9 definiert ist und/oder die Verwendung in einem Verfahren nach einem der Ansprüche 1 bis 14 erfolgt.

17. Flüssige Mischung, bevorzugt eine wässrige flüssige Mischung, zur beschleunigten Alterung eines Kunststoffartikels, umfassend
a) 0,03 bis 0,75 mol/L, bevorzugt 0,08 bis 0,4 mol/L, mindestens eines Oxidationskatalysators ausgewählt aus einem N-Hydroxyimid, insbesondere einem cyclischen N-Hydroxyimid, bevorzugt N-Hydroxyphthalimid und/oder N-Hydroxysuccinimid, und
b) 0,001 bis 0,1 mol/L, bevorzugt 0,005 bis 0,03 mol/L, mindestens eines Cokatalysators ausgewählt aus einem Übergangsmetallsalz oder einem Übergangsmetallkomplex, wobei das Übergangsmetall bevorzugt ausgewählt ist aus Cu, Mn, Co oder Fe, bevorzugter Co, und
c) 35 bis 80 Vol.-%, bevorzugt 45 bis 70 Vol.-%, mindestens einer Säure ausgewählt aus einer C₁₋₄-Carbonsäure, bevorzugt Ameisensäure und/oder Essigsäure.

## Claims

1. Method of accelerated ageing of a plastics article, comprising contacting the plastics article with a liquid mixture comprising
a) at least one oxidation catalyst and
b) optionally at least one cocatalyst for at least one oxidation catalyst and
c) at least one acid and
d) optionally water and/or at least one other solvent,
**characterized in that** the at least one oxidation catalyst is an N-hydroxyimide.

2. Method according to Claim 1, wherein the at least one oxidation catalyst is a cyclic N-hydroxyimide, preferably N-hydroxyphthalimide and/or N-hydroxysuccinimide.

3. Method according to either of the preceding claims, wherein the liquid mixture comprises:
a) at least one oxidation catalyst selected from an N-hydroxyimide, in particular a cyclic N-hydroxyimide, preferably N-hydroxyphthalimide and/or N-hydroxysuccinimide, and
b) at least one cocatalyst selected from a transition metal salt or a transition metal complex and
c) at least one acid selected from an inorganic acid or an organic acid and
d) optionally water and/or at least one other solvent.

4. Method according to any of the preceding claims, wherein the acid is sulfuric acid or at least one C₁₋₄ carboxylic acid.

5. Method according to any of the preceding claims, wherein the acid is selected from formic acid, acetic acid, propionic acid or a mixture thereof, preferably formic acid and/or acetic acid.

6. Method according to Claim 3, wherein the transition metal of the transition metal salt or transition metal complex is selected from Cu, Mn, Co or Fe, preferably Co.

7. Method according to any of the preceding claims,
wherein the liquid mixture comprises water, and/or
wherein the at least one oxidation catalyst selected from an N-hydroxyimide, and, if present, at least one cocatalyst, in particular the transition metal salt or the transition metal complex, are in dissolved form in the liquid mixture.

8. Method according to any of the preceding claims, wherein the liquid mixture, preferably the aqueous liquid mixture, contains
a) 0.03 to 0.75 mol/l, preferably 0.08 to 0.4 mol/l, of at least one oxidation catalyst selected from an N-hydroxyimide, in particular a cyclic N-hydroxyimide, preferably N-hydroxyphthalimide and/or N-hydroxysuccinimide, and
b) 0.001 to 0.1 mol/l, preferably 0.005 to 0.03 mol/l, of at least one cocatalyst selected from a transition metal salt or a transition metal complex, where the transition metal is preferably selected from Cu, Mn, Co or Fe, more preferably Co, and
c) 35% to 80% by volume, preferably 45% to 70% by volume, at least one acid selected from a C₁₋₄ carboxylic acid, preferably formic acid and/or acetic acid.

9. Method according to any of the preceding claims, wherein the plastic of the plastics article is a polycondensate or a polyadduct, wherein the plastic is preferably a polyester or a polyamide, preferably a polyamide.

10. Method according to any of the preceding claims,
wherein the plastics article is contacted with the liquid mixture at a temperature in the range from 15°C to 100°C, preferably in the range from 60°C to 90°C, more preferably from 70 to 85°C, and/or
wherein the plastics article is contacted with the liquid mixture over a period of 6 hours to 300 hours, preferably 20 hours to 80 hours.

11. Method according to any of the preceding claims, wherein an oxygen-containing gas, in particular air, is introduced into the liquid mixture during the ageing treatment.

12. Method according to any of the preceding claims, further comprising analysing the plastics article after it has been removed from the liquid mixture for determination of at least one property of the plastics article subjected to the ageing treatment, wherein the removed plastics article is optionally subjected to a reconditioning treatment before the analysis.

13. Method according to Claim 12, wherein the analysis of the plastics article comprises at least one of mechanical tensile testing, dynamic differential calorimetry, **IR** spectroscopy, optical analysis or a combination thereof.

14. Method according to any of the preceding claims, wherein the plastics article is contacted with the liquid mixture by dipping the plastics article into the liquid mixture or rinsing or flushing the plastics article with the liquid mixture or filling the plastics article with the liquid mixture.

15. Use of a liquid mixture as ageing medium for accelerated ageing of a plastics article, wherein the liquid mixture comprises
a) at least one oxidation catalyst and
b) optionally at least one cocatalyst for at least one oxidation catalyst and
c) at least one acid and
d) optionally water and/or at least one other solvent,
**characterized in that** the at least one oxidation catalyst is an N-hydroxyimide.

16. Use according to Claim 15, wherein the liquid mixture is as defined in any of Claims 2 to 8 and/or the plastics article is as defined in Claim 9 and/or the use is effected in a method according to any of Claims 1 to 14.

17. Liquid mixture, preferably an aqueous liquid mixture, for accelerated ageing of a plastics article, comprising
a) 0.03 to 0.75 mol/l, preferably 0.08 to 0.4 mol/l, of at least one oxidation catalyst selected from an N-hydroxyimide, in particular a cyclic N-hydroxyimide, preferably N-hydroxyphthalimide and/or N-hydroxysuccinimide, and
b) 0.001 to 0.1 mol/l, preferably 0.005 to 0.03 mol/l, of at least one cocatalyst selected from a transition metal salt or a transition metal complex, where the transition metal is preferably selected from Cu, Mn, Co or Fe, more preferably Co, and
c) 35% to 80% by volume, preferably 45% to 70% by volume, at least one acid selected from a C₁₋₄ carboxylic acid, preferably formic acid and/or acetic acid.

## Revendications

1. Procédé de vieillissement accéléré d'un article en matière plastique, comprenant la mise en contact de l'article en matière plastique avec un mélange liquide comprenant
a) au moins un catalyseur d'oxydation et
b) éventuellement au moins un co-catalyseur pour ledit au moins un catalyseur d'oxydation et
c) au moins un acide et
d) éventuellement de l'eau et/ou au moins un autre solvant,
**caractérisé en ce que** ledit au moins un catalyseur d'oxydation est un N-hydroxyimide.

2. Procédé selon la revendication 1, dans lequel ledit au moins un catalyseur d'oxydation est un N-hydroxyimide cyclique, de préférence le N-hydroxyphtalimide et/ou le N-hydroxysuccinimide.

3. Procédé selon l'une quelconque des revendications précédentes, dans lequel le mélange liquide comprend :
a) au moins un catalyseur d'oxydation choisi parmi un N-hydroxyimide, en particulier un N-hydroxyimide cyclique, de préférence le N-hydroxyphtalimide et/ou le N-hydroxysuccinimide, et
b) au moins un co-catalyseur choisi parmi un sel de métal de transition ou un complexe de métal de transition et
c) au moins un acide choisi parmi un acide inorganique ou un acide acide organique et
d) éventuellement de l'eau et/ou au moins un autre solvant.

4. Procédé selon l'une quelconque des revendications précédentes, dans lequel l'acide est l'acide sulfurique ou au moins un acide carboxylique en C₁₋₄.

5. Procédé selon l'une quelconque des revendications précédentes, dans lequel l'acide est choisi parmi l'acide formique, l'acide acétique, l'acide propionique ou un mélange de ceux-ci, de préférence l'acide formique et/ou l'acide acétique.

6. Procédé selon la revendication 3, dans lequel le métal de transition du sel de métal de transition ou complexe de métal de transition est choisi parmi Cu, Mn, Co ou Fe, de préférence Co.

7. Procédé selon l'une quelconque des revendications précédentes,
dans lequel le mélange liquide comprend de l'eau, et/ou
dans lequel ledit au moins un catalyseur d'oxydation choisi parmi un N-hydroxyimide, et, s'il est présent, ledit au moins un co-catalyseur, en particulier le sel de métal de transition ou le complexe de métal de transition, se trouvent sous forme dissoute dans le mélange liquide.

8. Procédé selon l'une quelconque des revendications précédentes, dans lequel le mélange liquide, de préférence le mélange liquide aqueux, contient
a) 0,03 à 0,75 mole/l, de préférence 0,08 à 0,4 mole/l, d'au moins un catalyseur d'oxydation choisi parmi un N-hydroxyimide, en particulier un N-hydroxyimide cyclique, de préférence le N-hydroxyphtalimide et/ou le N-hydroxysuccinimide, et
b) 0,001 à 0,1 mole/l, de préférence 0,005 à 0,03 mole/l, d'au moins un co-catalyseur choisi parmi un sel de métal de transition ou un complexe de métal de transition, le métal de transition étant choisi de préférence parmi Cu, Mn, Co ou Fe, de préférence Co, et
c) 35 à 80 % en volume, de préférence 45 à 70 % en volume, d'au moins un acide choisi parmi un acide carboxylique en C₁₋₄, de préférence l'acide formique et/ou l'acide acétique.

9. Procédé selon l'une quelconque des revendications précédentes, dans lequel la matière plastique de l'article en matière plastique est une matière plastique polycondensat ou une matière plastique polymère d'addition, la matière plastique étant de préférence un polyester ou un polyamide, de préférence un polyamide.

10. Procédé selon l'une quelconque des revendications précédentes,
dans lequel la mise en contact de l'article en matière plastique avec le mélange liquide s'effectue à une température dans la plage de 15 °C à 100 °C, de préférence dans la plage de 60 °C à 90 °C, de préférence de 70 à 85 °C, et/ou
dans lequel la mise en contact de l'article en matière plastique avec le mélange liquide s'effectue pendant une durée de 6 heures à 300 heures, de préférence 20 heures à 80 heures.

11. Procédé selon l'une quelconque des revendications précédentes, dans lequel pendant le traitement de vieillissement un gaz contenant de l'oxygène, en particulier de l'air, est introduit dans le mélange liquide.

12. Procédé selon l'une quelconque des revendications précédentes, comprenant en outre l'analyse de l'article en matière plastique après qu'il a été retiré du mélange liquide, pour la détermination d'au moins une propriété de l'article en matière plastique soumis au traitement de vieillissement, l'article en matière plastique retiré étant éventuellement soumis à un traitement de reconditionnement avant l'analyse.

13. Procédé selon la revendication 12, dans lequel l'analyse de l'article en matière plastique comprend au moins un(e) parmi essai de traction mécanique, calorimétrie différentielle dynamique, spectroscopie IR, analyse optique ou une combinaison de telles analyses.

14. Procédé selon l'une quelconque des revendications précédentes, dans lequel l'article en matière plastique est mis en contact avec le mélange liquide par immersion de l'article en matière plastique dans le mélange liquide, ou rinçage ou rinçage à fond de l'article en matière plastique avec le mélange liquide, ou remplissage de l'article en matière plastique avec le mélange liquide.

15. Utilisation d'un mélange liquide en tant que milieu de vieillissement pour le vieillissement accéléré d'un article en matière plastique, le mélange liquide comprenant
a) au moins un catalyseur d'oxydation et
b) éventuellement au moins un co-catalyseur pour ledit au moins un catalyseur d'oxydation et
c) au moins un acide et
d) éventuellement de l'eau et/ou au moins un autre solvant,
**caractérisé en ce que** ledit au moins un catalyseur d'oxydation est un N-hydroxyimide.

16. Utilisation selon la revendication 15, dans laquelle le mélange liquide est tel que défini dans l'une quelconque des revendications 2 à 8 et/ou l'article en matière plastique est tel que défini dans la revendication 9 et/ou l'utilisation s'effectue dans un procédé selon l'une quelconque des revendications 1 à 14.

17. Mélange liquide, de préférence un mélange liquide aqueux, destiné au vieillissement accéléré d'un article en matière plastique, comprenant
a) 0,03 à 0,75 mole/l, de préférence 0,08 à 0,4 mole/l, d'au moins un catalyseur d'oxydation choisi parmi un N-hydroxyimide, en particulier un N-hydroxyimide cyclique, de préférence le N-hydroxyphtalimide et/ou le N-hydroxysuccinimide, et
b) 0,001 à 0,1 mole/l, de préférence 0,005 à 0,03 mole/l, d'au moins un co-catalyseur choisi parmi un sel de métal de transition ou un complexe de métal de transition, le métal de transition étant choisi de préférence parmi Cu, Mn, Co ou Fe, de préférence Co, et
c) 35 à 80 % en volume, de préférence 45 à 70 % en volume, d'au moins un acide choisi parmi un acide carboxylique en C₁₋₄, de préférence l'acide formique et/ou l'acide acétique.
